# EUROPEAN PATENT APPLICATION

(11) **EP 1 946 751 A1**
(43) Date of publication of application: **23.07.2008**
(21) Application number: 07001304.0
(22) Date of filing: 22.01.2007
(51) Int. Cl.: A61K 9/68, A61P 1/02

(54) **Carrageenan-based chewing gum**

(71) Applicant: Documedica S.A., 6900 Lugano-Paradiso (CH)
(72) Inventor: Salini, Alberto, 6900 Lugano-Paradiso (CH)
(74) Representative: Minoja, Fabrizio

(57) **Abstract**

Chewing gums containing carrageenans as active ingredient.

## Description

The present invention relates to carrageenan-based compositions in chewing gum for the treatment of xerostomia.

### Background to the invention

Sufficient production of saliva is essential to the health. Saliva performs important physiological functions such as forming and moving the food bolus, lubricating the oral mucosa and keeping it intact, preventing demineralisation and promoting remineralisation of the teeth.

Saliva contains many antimicrobial proteins which control bacterial, fungal and viral growth.

Saliva also buffers the acid substances produced by bacteria, cleans the mouth after meals, and helps to preserve flavour and retain the dentures.

Saliva production by the submandibular and sublingual glands is moderately reduced in healthy elderly people, but this change is probably not biologically significant.

Xerostomia (also known as aptyalia or dry mouth) is a disorder caused by salivary hypoproduction secondary to dehydration, which has a number of iatrogenic and pathological causes.

It can be a side effect of over 400 drugs, many of which are commonly used, for example by elderly people, such as anticholinergics, antidepressants, antihistamines, antihypertensives (ACE-inhibitors, beta-blockers, calcium antagonists and diuretics), diuretics, antireflux drugs, antiparkinson drugs, antipsychotics and tranquillisers (particularly benzodiazepines); especially drugs with anticholinergic or diuretic properties.

External radiotherapy performed, for example, for tumours of the head and neck, destroys the tissue of the salivary glands and causes xerostomia in the long term; anti-tumoral chemotherapy with cytotoxic drugs can also adversely affect the function of the salivary glands.

One of the pathological causes is Sjögren's syndrome, the most common disorder of the salivary glands, which causes xerostomia and mainly affects post-menopausal women.

Other disorders of the salivary glands which cause xerostomia are obstructions of the ducts (e.g. sialolithiasis, mucoceles), infections (e.g. acute or chronic viral or bacterial sialadenitis) and tumours (e.g. pleomorphic adenomas, adenoid cystic carcinoma, adenocarcinomas and acinar-cell carcinoma).

Alteration of the salivary gland function can also occur in patients suffering from Alzheimer's disease, diabetes, sarcoidosis, cystic fibrosis and AIDS.

Even when the salivary gland function is normal, xerostomia may occur, for example when the oral sensory receptors are defective, or due to altered cognitive capacity.

Xerostomia can also be caused by dialysis treatment.

Xerostomia causes considerable discomfort to the patient, and can lead to the appearance of serious pathological conditions in time. Patients with hypofunction of the salivary glands typically have difficulty swallowing food, especially dry foods, and need to drink in order to swallow. The lips and mouth become dry with chewing; other symptoms are speech difficulty, halitosis and poor oral hygiene, because the reduction in salivary flow does not keep bacteria away for a sufficiently long time.

To alleviate the problems caused by xerostomia, at least temporarily, it can be useful to drink frequently and chew gums containing xylitol, for example; saliva substitutes, such as compositions based on cellulose derivatives like carboxymethylcellulose, are partly useful, but the results are only modest.

### Description of the invention

It has now surprisingly been found that carrageenan-based compositions in chewing gum provide marked, lasting relief of the symptoms caused by xerostomia, and effectively prevent lesions of the oral epithelium.

Carrageenans are natural polysaccharides, with particular chemico-physical characteristics and special rheological properties which differentiate them substantially from other biopolymers, such as cellulose and derivatives thereof, pectins, alginates, etc.

Carrageenans are widely used for their viscosity-controlling, gelling, stabilising and emollient properties in the pharmaceutical, cosmetic and food industries.

This invention includes all compositions containing carrageenan in chewing gum which are made by known methods.

The compositions in the form of chewing gum, for example, are those described in US 3,262,784, in the form of chewable tablets.

Alternatively, the chewing gum containing carrageenans can take the form of multilayer chewable tablets as described, for example, in WO 2004/073691.

In the compositions of the invention, the quantity of carrageenans is between 1 mg and 300 mg per dose; the concentration is preferably between 5 mg and 100 mg per dose.

Natural or synthetic compounds with activity complementary to the treatment of xerostomia, such as bacteriostatics, synthetic and natural bactericides, anti-inflammatories, wound-healing compounds, antivirals, antifungals, biopolymers, mineral compounds such as fluorides, plant extracts such as essential oils, mother tinctures, etc., could be added to the compositions according to this invention.

The examples below illustrate the invention in greater detail.

### EXAMPLE 1

Chewing gum tablets are prepared by the process described in example 1 of US 3,262,784; one tablet contains 10 mg of carrageenans.

Tablets containing 5 mg and 100 mg of carrageenans per tablet are obtained with the same process.

### EXAMPLE 2

Chewing gum tablets are prepared by the process described in example 1 of WO 2004/073691; one tablet contains 20 mg of carrageenans.

Tablets containing 5 mg and 100 mg of carrageenans are obtained with the same process.

### EXAMPLE 3

Chewing gum tablets with the following composition are prepared by the process described in example 1 of WO 2004/073691:

| | |
|---|---|
| Sorbitol (sweetener) | 38.087% |
| Gum base (chewing medium) | 30.912% |
| Isomalt (sweetener) | 20.835% |
| Talc (anti-caking agent) | 3.404% |
| Silicon dioxide (anti-caking agent) | 1.935% |
| Vegetable magnesium stearate (anti-caking agent) | 1.715% |
| Flavourings | 1.696% |
| Carrageenan (active ingredient) | 1.176% |
| Menthol (flavouring) | 0.118% |
| Acesulfame K (sweetener) | 0.050% |
| Saccharine sodium salt (sweetener) | 0.050% |
| E 132 (colouring) | 0.022% |
| 1 tablet = 1.700 mg | |
| Dose: 3 - 4 tablets/day or as prescribed by the physician. | |

### EXAMPLE 4

Chewing gum tablets with the following composition are prepared by the process described in example 1 of WO 2004/073691:

| | |
|---|---|
| Gum base (chewing medium) | 43.960% |
| Isomalt (sweetener) | 29.630% |
| Sorbitol (sweetener) | 13.556% |
| Talc (anti-caking agent) | 4.841 % |
| Vegetable magnesium stearate (anti-caking agent) | 2.270% |
| Silicon dioxide (anti-caking agent) | 2.218% |
| Flavourings | 2.187% |
| Carrageenan (active ingredient) | 1.111 % |
| Menthol (flavouring) | 0.111 % |
| Saccharine sodium salt (sweetener) | 0.047% |
| Acesulfame K (sweetener) | 0.047% |
| E 132 (colouring) | 0.021% |
| 1 tablet = 1.700 mg | |
| Dose: 3 - 4 tablets/day or as prescribed by the physician. | |

### EXAMPLE 5

Chewing gum tablets with the following composition are prepared by the process described in example 1 of WO 2004/073691:

| | |
|---|---|
| Sorbitol (sweetener) | 38.028% |
| Gum base (chewing medium) | 30.874% |
| Isomalt (sweetener) | 20.809% |
| Talc (anti-caking agent) | 3.400% |
| Silicon dioxide (anti-caking agent) | 1.934% |
| Vegetable magnesium stearate (anti-caking agent) | 1.713% |
| Mint flavouring | 1.224% |
| Carrageenan (active ingredient) | 1.176% |
| Eucalyptus flavouring | 0.471% |
| Aspartame (sweetener) | 0.194% |
| Menthol (flavouring) | 0.118% |
| Acesulfame K (sweetener) | 0.036% |
| E 132 (colouring) | 0.022% |
| 1 tablet = 1.700 mg | |
| Dose: 3 - 4 tablets/day or as prescribed by the physician. | |

## Claims

1. Chewing gums containing carrageenans as active ingredient.

2. Chewing gums as claimed in claim 1, in the form of a chewable tablet.

3. Chewing gums as claimed in claim 1, in the form of a multilayer chewable tablet.

4. Use of carrageenans to prepare chewing gums for the treatment of xerostomia.
